# EUROPEAN PATENT APPLICATION

(11) **EP 4 834 280 A1**
(43) Date of publication of application: **04.11.2026**
(21) Application number: 24911242.6
(22) Date of filing: 25.12.2024
(51) Int. Cl.: A61F 2/07, A61F 2/82

(54) **COVERED STENT**

(30) Priority: 29.12.2023 CN 202311863063
(71) Applicant: Lifetech Scientific (Shenzhen) Co., Ltd., Shenzhen, Guangdong 518063 (CN)
(72) Inventor: TANG, Chunwei, Shenzhen, Guangdong 518063 (CN); WU, Xuan, Shenzhen, Guangdong 518063 (CN)
(74) Representative: Michalski Hüttermann & Partner mbB
(86) International application number: PCT/CN2024/142341
(87) International publication number: WO 2025/140319

(57) **Abstract**

The present application relates to a covered stent, which includes a main stent and a branch stent, wherein the main stent includes a branch opening communicated with the branch stent; the branch stent includes a first end and a second end which are opposite to each other, the first end being connected to the branch opening; and the branch stent includes an adjustment section close to the first end and a support section close to the second end, the adjustment section including a reinforcing rib, to allow the support section to be driven to move downward when the branch stent is subjected to force. In the covered stent according to the present application, the reinforcing rib is arranged at the adjustment section to allow the support section to be driven to move downward when the branch stent is subjected to force. Therefore, the present application provides the covered stent that can be flexibly adjusted and positioned and reduce the required positioning accuracy, without affecting blood flow in the aorta and branch arteries or interfering with the construction of a branch guidewire.

## Description

### Technical

The embodiments relate to the technical field of medical devices, and in particular, to a covered stent.

### Background

Branch stent reconstruction has become one of the mainstream intraluminal branch reconstruction techniques. However, the current branch stent reconstruction technique faces the following two major challenges. First, a stent with branches requires high positioning accuracy. Second, although a stent with internal branches can reduce the required positioning accuracy when connecting an external branch stent, the internal branch stents occupy space within a main lumen, which affects the hemodynamics in the main lumen.

### Summary

To solve the technical problems, the embodiments provide a covered stent, which can be flexibly adjusted and positioned and reduce the required positioning accuracy, without affecting blood flow in the aorta and branch arteries or interfering with the construction of a branch guidewire.

The embodiments provide a covered stent. The covered stent includes a main stent and a branch stent, the main stent includes a branch opening communicated with the branch stent, the branch stent includes a first end and a second end which are opposite to each other, the first end is connected to the branch opening, the branch stent includes an adjustment section close to the first end and a support section close to the second end, and the adjustment section includes a reinforcing rib, to allow the support section to be driven to move downward when the branch stent is subjected to force.

In one embodiment, the adjustment section further includes a connecting film which connects the branch opening and the support section, the reinforcing rib is arranged on the connecting film, and the reinforcing rib is a flexible reinforcing rib.

In one embodiment, the reinforcing rib has a "chain-like" discontinuous point structure or discontinuous segment structure.

In one embodiment, the reinforcing rib includes a first section and a second section in an extension direction of the reinforcing rib, and a gap is formed between the first section and the second section.

In one embodiment, the reinforcing rib includes a suture line sutured along the connecting film;
alternatively, the connecting film includes a folded portion, and the folded portion is thermally laminated to form the reinforcing rib; and
alternatively, the connecting film includes a folded portion, and the reinforcing rib includes the folded portion and a suture line, and the suture line is integrally sutured along the folded portion to form the reinforcing rib.

In one embodiment, at least two reinforcing ribs are arranged at intervals in a circumferential direction of the branch stent.

In one embodiment, the branch stent includes a first circumferential support member arranged in a circumferential direction of the branch stent, and the first circumferential support member is arranged at a connection portion of the adjustment section and the support section.

In one embodiment, the branch stent further includes a second circumferential support member and a third circumferential support member, the second circumferential support member is arranged at the first end, and the third circumferential support member is arranged at the second end.

In one embodiment, a diameter of the first circumferential support member is defined as D1, a diameter of the second circumferential support member is defined as D2, and a diameter of the third circumferential support member is defined as D3, and D1, D2, and D3 satisfy: D1≤D3<D2; and
alternatively, D1, D2, and D3 satisfy: D1≤D3, and 1.5D1≤D2≤3D1.

In one embodiment, the reinforcing rib includes a first end portion and a second end portion, the first end portion is close to the first end, the second end portion is close to the second end, a gap is formed between the first end portion and the first end of the branch stent, or/and a gap is formed between the second end portion and the adjustment section.

A technical effect of one embodiment of the present application is that the reinforcing rib is arranged at the adjustment section to allow the support section to be driven to move downward when the branch stent is subjected to force. Therefore, the present application provides the covered stent that can be flexibly adjusted and positioned and reduce the required positioning accuracy, without affecting blood flow in the aorta and branch arteries or interfering with the construction of a branch guidewire.

### Brief Description of the Drawings

FIG. 1 is a schematic structural diagram of a covered stent provided by the present application;
FIG. 2 is a schematic structural diagram of a branch stent provided by the present application;
FIG. 2a is a schematic structural diagram of another branch stent provided by the present application;
FIG. 2b is a schematic structural diagram of yet another branch stent provided by the present application;
FIG. 3 is a top view of FIG. 2;
FIG. 4 is a schematic structural diagram of another branch stent provided by the present application;
FIG. 5 is a schematic diagram of a branch stent of the covered stent provided in FIG. 1 moving downward into a lumen of a main stent after the branch stent is subjected to force shown as F1 in FIG. 1;
FIG. 6 is a schematic diagram of a branch stent of the covered stent provided in FIG. 1 moving downward into a lumen of a main stent after the branch stent is subjected to force shown as F2 in FIG. 1;
FIG. 7 is a schematic structural diagram of another branch stent provided by the present application;
FIG. 8 is an enlarged view of part A in FIG. 7;
FIG. 9 is an enlarged view of part A in FIG. 7 in another implementation;
FIG. 10 is a top view of FIG. 9;
FIG. 11 is an enlarged view of part A in FIG. 7 in another implementation;
FIG. 12 is a top view of FIG. 11;
FIG. 13 is a top view of a branch stent provided by the present application (viewed from a second end to a first end of the branch stent);
FIG. 14 is a schematic diagram showing a first section of the branch stent provided in FIG. 13 in a folded-down state;
FIG. 15 is a schematic diagram based on FIG. 14, with a second section folded down;
FIG. 16 is a schematic diagram based on FIG. 14, showing a support section tilting relative to a second section;
FIG. 17 is a schematic diagram based on FIG. 14, showing a micro "cradle-type" structure translating and swinging toward a left side;
FIG. 17a is a top view of a branch stent in FIG. 17;
FIG. 18 is a schematic diagram based on FIG. 14, showing a micro "cradle-type" structure translating and swinging toward a right side;
FIG. 18a is a top view of a branch stent in FIG. 18;
FIG. 19 is a schematic structural diagram of an aortic arch stent provided by the present application;
FIG. 20 is a schematic structural diagram of a thoracoabdominal main stent provided by the present application; and
FIG. 21 is a schematic structural diagram of an abdominal main stent provided by the present application.

100: covered stent; 10: main stent; 11: branch opening; 12: first bare corrugated ring; 13: main corrugated ring; 14: main covering film; 20: branch stent; 20a: first end; 20b: second end; 21: adjustment section; 211: connecting film; 2111: folded portion; 212: reinforcing rib; 212a: first section; 212b: second section; 2121: first end portion; 2122: second end portion; 22: support section; 22a: proximal portion; 22b: distal portion; 221: support covering film; 222: support corrugated ring;
23: first circumferential support member; 24: second circumferential support member; and 25: third circumferential support member.

### Detailed Description of the Invention

For the purpose of understanding the present application, a comprehensive description will be made to the present application below with reference to the relevant drawings. The drawings show preferred implementations of the present application. However, the present application can be implemented in various forms and should not be limited by the implementations set forth herein. On the contrary, these implementations are provided to enable a more thorough understanding of the present application.

It should be noted that when an element is referred to as being "secured" to another element, element may be directly secured to the another element or indirectly secured via an intermediate element. When an element is referred to as being "connected" to another element, the element may be directly connected to the another element or indirectly connected via an intermediate element. Terms "inner", "outer", "left", "right", and other similar expressions used in the text are only for illustration, and do not represent a unique implementation. An axial direction of a branch stent defined in the present application refers to an extension direction along the tubular branch stent, and a radial direction of the branch stent is perpendicular to its axial direction; and an axial direction of a main stent refers to an extension direction along the tubular main stent.

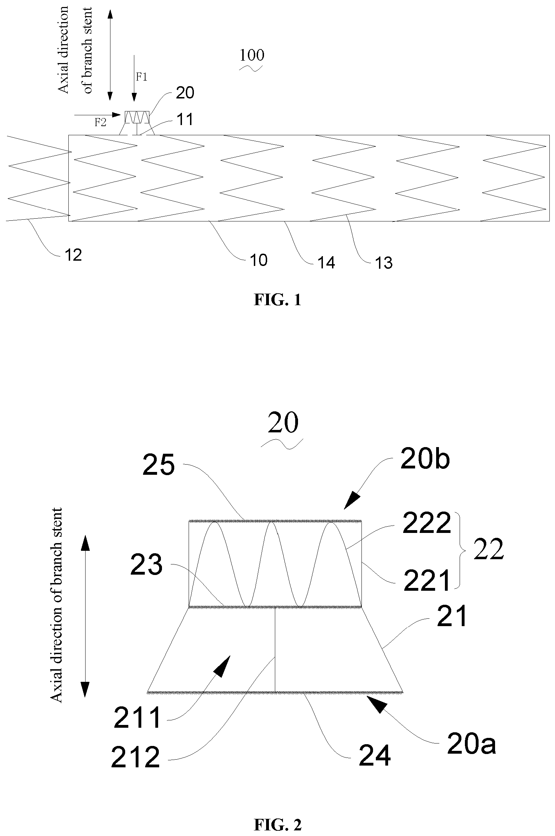

The present application provides a covered stent 100. As shown in FIG. 1, the covered stent 100 includes a main stent 10 and a branch stent 20. The main stent 10 is a tubular structure with openings at both ends, and includes a first bare corrugated ring 12, main corrugated rings 13, and a main covering film 14. A plurality of main corrugated rings 13 are arranged, the plurality of main corrugated rings 13 are arranged in an axial direction of the main stent 10 and are connected through the tubular main covering film 14, a number of main corrugated rings 13 may be set reasonably according to an axial length of the main stent 10 in combination with a wave height of the corrugated rings, and the main corrugated rings 13 are kept away from a branch opening 11, such that the branch stent 20 is conveniently connected to the branch opening 11, and a main lumen of the main stent 10 is communicated with a lumen of the branch stent 20.

The axial length of the main stent 10 can be set in a range from 40 mm to 240 mm, and the first bare corrugated ring 12 is arranged at a proximal end of the main corrugated ring 13, and is connected to an edge of a proximal end of the main covering film 14. In other implementations, the first bare corrugated ring 12 may not be arranged, and at this time, a proximal end of the covering film is a proximal end of the main stent 10.

In this implementation, the main corrugated ring 13 is arranged on an outer side of the main covering film 14, and during implantation in a blood vessel, frictional force between the main stent 10 and an inner wall of the blood vessel can be increased, thereby helping to prevent displacement or shortening of the main stent 10 relative to the inner wall of the blood vessel. In other implementations, the main corrugated ring 13 may also be arranged on an inner side of the main covering film 14, or the main corrugated ring 13 is partially arranged on an inner side of the main covering film 14 and partially arranged on an outer side of the main covering film 14, which is not limited herein.

As shown in FIG. 1 to FIG. 2, the branch stent 20 includes a first end 20a and a second end 20b which are opposite to each other, the first end 20a can be sutured and connected to the branch opening 11 of the main stent 10 through a suture line in a circumferential direction of the branch stent. The branch stent 20 further includes an adjustment section 21 and a support section 22, the adjustment section 21 is close to the first end 20a of the branch stent 20, and the support section 22 is close to the second end 20b of the branch stent 20. The support section 22 includes a support covering film 221 and a support corrugated ring 222, the support covering film 221 covers an inner side or an outer side of the support corrugated ring 222, an edge, close to a first section 212a, of the support corrugated ring 222 is flush or substantially flush with an edge of the support covering film 221 on a same side, an edge, close to the second end 20b, of the support corrugated ring 222 is flush or substantially flush with the edge of the support covering film 221 on the same side, and substantially flush refers to edge misalignment of not more than 1 mm, such that the support corrugated ring 222 has a better supporting effect on the edge of the support section 22.

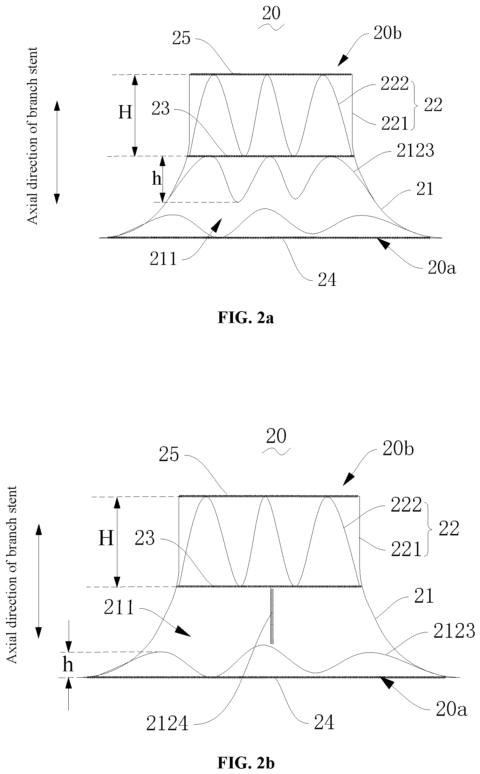

As shown in FIG. 2 to FIG. 3, the adjustment section 21 includes a connecting film 211 and a reinforcing rib 212 arranged on the connecting film 211. In this implementation, the adjustment section 21 is connected to the support section 22, and the connecting film 211 connects the branch opening 11 and the support section 22, wherein the support covering film 221 is connected to the connecting film 211 to form a branch covering film of the branch stent 20, the support corrugated ring 222 includes at least one corrugated annular element, and the corrugated annular element extends circumferentially in a wave-like manner around the tubular support covering film 221 (i.e. around the branch stent 20) for a full turn, to support the support covering film 221 and maintain the lumen of the branch stent 20. In this implementation, a length of the branch stent 20 in its axial direction ranges from 5 mm to 40 mm, wherein a length of the adjustment section 21 in the axial direction of the branch stent 20 ranges from 3 mm to 20 mm; and a length of the support section 22 in the axial direction of the branch stent 20 ranges from 2 mm to 20 mm.

In this implementation, the reinforcing rib 212 extends from the first end 20a to the second end 20b on the connecting film 211, or the reinforcing rib 212 extends from the second end 20b to the first end 20a, and an extension direction herein only indicates a direction of extension of the reinforcing rib 212 in length, rather than a starting or ending direction of extension of the reinforcing rib 212, such that when the second end 20b of the branch stent 20 or a side edge of the branch stent 20 is subjected to force, the reinforcing rib 212 provides support for the connecting film 211, and the reinforcing rib 212 rotates relative to the first end 20a to drive the support section 22 to at least partially move downward into the adjustment section 21. In addition, a condition that the connecting film 211 is too soft to provide support in the axial direction of the branch stent 20 to cause stacking of the connecting film 211 in the axial direction is prevented. If the connecting film 211 of the adjustment section 21 is stacked in the axial direction of the branch stent 20, during the construction of a branch guidewire, the stacked connecting film 211 tends to impede the advancement of the branch guidewire.

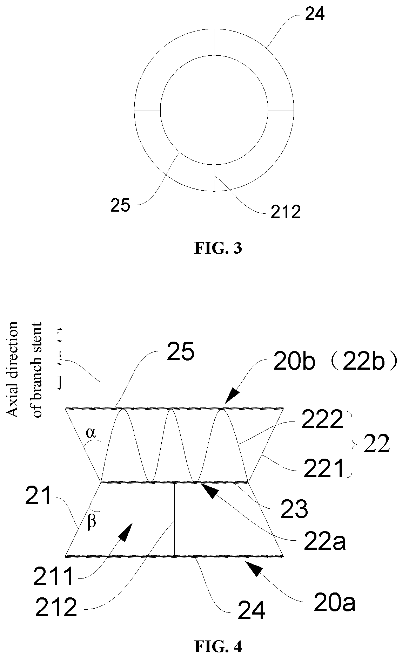

In other implementations, except two ends connected to the connecting film, other portions of the reinforcing rib may be suspended from the connecting film, and the reinforcing rib may also support the connecting film in the axial direction of the branch stent, to facilitate rotation of the reinforcing rib relative to the first end, which is not limited herein. As shown in FIG. 2 to FIG. 3, the support section 22 may have an isodiametric tubular structure, and the support section 22 includes a distal portion 22b away from the main stent 10 and a proximal portion 22a close to the main stent 10, wherein the distal portion 22b and the proximal portion 22a are concentric and isodiametric to enable the support section 22 to form an isodiametric lumen. In other implementations, the support section 22 may also have a non-isodiametric tubular structure; when the support section 22 has the non-isodiametric tubular structure, an annular opening diameter of the proximal portion 22a of the support section 22 is less than or equal to an annular opening diameter of the distal portion 22b (i.e. an end portion of the second end 20b of the branch stent 20) of the support section 22; as shown in FIG. 4, the support section 22 can be of an inverted circular truncated cone structure of which a diameter gradually increases from the proximal portion 22a to the distal portion 22b, and an axial angle α between a side edge of the support section 22 and the branch stent 20 is less than or equal to an axial angle β between a side edge of the adjustment section 21 and the branch stent 20, such that after the support section 22 moves downward into the folded-down adjustment section 21, sufficient space remains in the folded-down adjustment section 21 to allow the support section 22 to tilt toward the circumferential direction and flexibly swing; and the support section 22 may also have a drum-shaped structure with a large-diameter middle part and two small-diameter ends, thereby facilitating downward movement of the support section 22 relative to the adjustment section 21 and facilitating flexible rotation of the support section 22 in the circumferential direction of the branch stent 20.

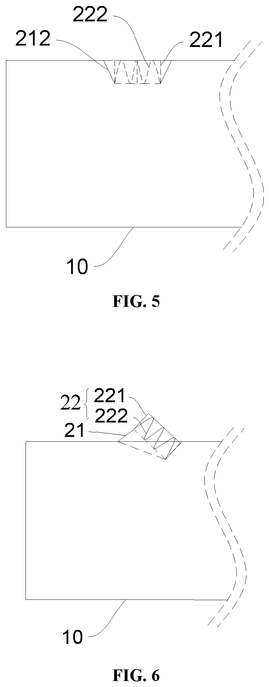

As shown in FIG. 2 to FIG. 3 and FIG. 5 in combination with Fig. 1, when the second end 20b of the branch stent 20 is subjected to force from top to bottom in FIG. 1, a direction of the force is as shown by an arrow F1 in FIG. 1, and the reinforcing rib 212 of the adjustment section 21 rotates relative to the first end 20a, such that the adjustment section 21 rotates from an outer side of the main stent 10 to an inner side of the main stent 10, thereby driving the support section 22 to move downward into the lumen of the main stent 10 as a whole, as shown in FIG. 5. Because the support section 22 which moves downwards can flexibly swing, when the branch guidewire passes through the support section 22 which moves downward, according to an advancing direction of the branch guidewire, the support section 22 can be pulled by the branch guidewire and tilt toward a branch vessel opening. In this implementation, heights of the adjustment section 21 and the support section 22 in the axial direction of the branch stent 20 may also be set to be equal, and when the adjustment section 21 is folded inward toward the lumen of the main stent 10 and drives the support section 22 to move downward at the same time, the second end 20b of the branch stent 20 just moves downward to be flush with a side edge of the main stent 10, as shown in FIG. 5. In other implementations, a height of the adjustment section 21 in the axial direction of the branch stent 20 may be set to be greater than a height of the support section 22 in the axial direction of the branch stent 20, such that the branch stent 20 moves downward into the lumen of the main stent 10, and a condition that the branch stent 20 is pressed due to narrow space of a true lumen of an aorta can be avoided.

As shown in FIG. 6 in combination with FIG. 1, when the branch stent 20 is subjected to lateral force as shown in a direction of an arrow F2 in FIG. 1, the reinforcing rib 212 on an opposite side of a force bearing point rotates relative to a corresponding position of the first end 20a, and the adjustment section 21 on the opposite side of the force bearing point (a right side of the branch stent 20 in FIG. 1) drives the support section 22 on the side to move downward, such that the adjustment section 21 tilts toward the opposite side (the right side) of the force bearing point (a left side of the branch stent 20 in FIG. 1), as shown in FIG. 6. When the second end 20b or the side edges of the branch stent 20 are subjected to an external force, no circumferential corrugated supporting members are disposed on the adjustment section 21, so that the adjustment section 21 is allowed to move downward(which is distinct from the prior art where the adjustment section is provided with circumferential corrugated supporting members and configured as a shaped tube, resulting in inability to move downward), the reinforcing rib 212 extends in this direction, and, in combination with flexibility of the connecting film 211, the adjustment section 21 can be at least partially folded into the lumen of the main stent 10 due to force, to facilitate adjustment of a direction of an end opening of the second end 20b of the branch stent 20, or the support section 22 is allowed to move downward in the axial direction of the branch stent 20 to approach the main stent 10 and even entirely move downward into the lumen of the main stent 10. Due to its self-supporting nature, the support section 22 maintains its original shape and is only positioned close to the lumen of the main stent 10.

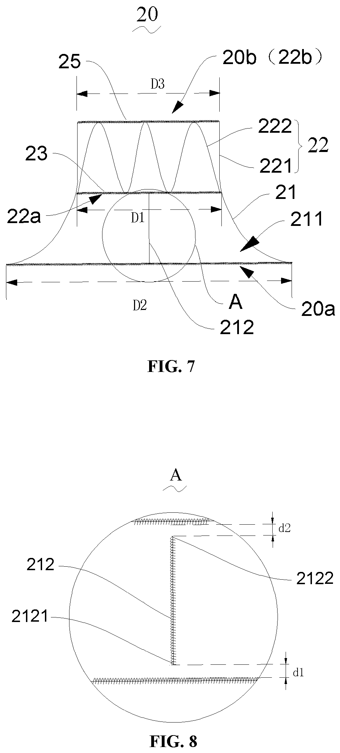

As shown in FIG. 7, the branch stent 20 includes a first circumferential support member 23 arranged in the circumferential direction of the branch stent 20, and the first circumferential support member 23 is arranged at a connection portion between the adjustment section 21 and the support section 22. In this implementation, the first circumferential support member 23 may be formed by suturing in the circumferential direction of the connection portion through a suture line, the support covering film 221 and the connecting film 211 may be two sections of separate films and connected by suturing through a suture line, or may be an integrated film, and then sutured using a suture line in the circumferential direction at a boundary of the support covering film and the connecting film. The first circumferential support member 23 may be sutured continuously in the circumferential direction, or sutured interruptedly, which is not limited herein, as long as a support rather than the films is formed by suturing in the circumferential direction at the connection portion. In other implementations, the first circumferential support film 23 may be a plurality of discontinuous developing arcs arranged at intervals in the circumferential direction, or a continuous developing ring, and the developing arcs or the developing ring is then sutured onto the connection portion, to provide support other than the films at the connection portion. When the circumferential support member is discontinuous arcs, its diameter is a diameter of a circle corresponding to the arcs. The branch stent 20 further includes a second circumferential support member 24 and a third circumferential support member 25, the second circumferential support member 24 is arranged at the first end 20a of the branch stent 20 (i.e. an end portion of the adjustment section 21 away from the support section 22), and the third circumferential support member 25 is arranged at the second end 20b of the branch stent 20 (i.e. an end portion of the support section 22 away from the adjustment section 21, that is, the distal portion 22b of the support section 22); and a diameter of the first circumferential support member 23 is defined as D1, a diameter of the second circumferential support member 24 is defined as D2, and a diameter of the third circumferential support member 25 is defined as D3, wherein D1, D2, and D3 satisfy: D1≤D3<D2. This configuration facilitates the inward folding of the adjustment section 21 toward the lumen of the main stent 10, which in turn allows the support section 22 to move downward. It also facilitates the support section 22 which moves downward to swing freely in the circumferential direction of the branch stent 20 relative to the first circumferential support member 23. The first circumferential support member 23 is arranged to have circumferential support force between the adjustment section 21 and the support section 22. The first circumferential support member 23 is equivalent to preset a crease between the adjustment section 21 and the support section 22, when the branch stent 20 is subjected to force, relative folding between the adjustment section 21 and the support section 22 is more simple and the connecting film 211 is prevented from being stacked when the adjustment section 21 is turned over in a direction close to the main stent 10, such that a folding position of the adjustment section 21 close to one side of the support section 22 is controllable; the adjustment section 21 is folded in a direction close to the main stent 10 with the first end 20a as a fulcrum, such that one end of the adjustment section 21 close to the support section 22 moves downward, thereby driving the support section 22 to move downward; and in addition, in respect of a relative position, the adjustment section 21 is folded toward the support section 22 relative to the connection portion (the first circumferential support member 23) between the adjustment section and the support section. The first circumferential support member 23 can better maintain an annular form of the proximal portion 22a of the support section in the process of driving the support section 22 to move downward, and prevent the annular form of the proximal portion of the support section from being affected due to no corrugated rings for supporting the adjustment section, thereby facilitating reconstruction of the branch guidewire. The second circumferential support member 24 and the third circumferential support member 25 also increase support for the first end 20a and the second end 20b of the branch stent 20, respectively. In other implementations, any of the first circumferential support member 23, the second circumferential support member 24, and the third circumferential support member 25 may be an entire circumferential continuous ring or a plurality of arcs arranged at intervals, which may be sutured and wrapped in the covering film through a suture line, to support a covering film opening in a ring shape.

The first circumferential support member 23, the second circumferential support member 24, and the third circumferential support member 25 may also be configured as a developing material, such as a tantalum wire, etc., such that the folded-down adjustment section 21 and the support section 22 which moves downward form the branch stent 20 more easily in a body to provide a good developing property, and super-selective reconstruction of branches is facilitated. In this implementation, when the first circumferential support member 23 and the third circumferential support member 25 are arranged, two ends of the support corrugated ring 222 in the axial direction of the branch stent 20 can each abut against the circumferential support member on the same side thereof, to better maintain a stent form of the support section 22, as shown in FIG. 7.

In this implementation, as shown in FIG. 2 in combination with FIG. 7, the support section 22 has an isodiametric tubular structure, i.e. D1=D3. The adjustment section 21 has a hollow circular truncated cone-like shape, that is, D1<D2, and projections of the first circumferential support member 23 and the third circumferential support member 25 on a radial cross section of the branch stent 20 do not exceed a projection of the second circumferential support member 24 on the radial cross section of the branch stent 20, such that the adjustment section 21 is not prevented when folded toward the lumen of the main stent 10, to smoothly drive the support section 22 to move downward, and convenience is provided for the support section 22 which moves downward to swing freely relative to the first circumferential support member 23 in the circumferential direction of the branch stent 20. Further, D1 and D2 also satisfy: 1.5D1≤D2≤3D1; when a size difference between the two ends of the adjustment section 21 is too small, it may result in a condition that the support section 22 is inconveniently folded toward the lumen of the main stent 10 relative to the first end 20a of the branch stent 20; and when the size difference between the two ends of the adjustment section 21 is too large, it is inconvenient to design the shape of the adjustment section 21. When D1 and D2 satisfy: 1.5D1≤D2≤3D1, it is more conducive to shape design of the adjustment section 21, and folding of the adjustment section 21 toward the lumen of the main stent 10 when the branch stent 20 is subjected to an external force.

In this implementation, four reinforcing ribs 212 are uniformly arranged in the circumferential direction of the branch stent 20, as shown in FIG. 3, and a central angle spanned between two adjacent reinforcing ribs 212 is 90°, such that the support section 22 which moves downward can swing freely within a range of 360° around the axial direction of the branch stent 20 (swing freely along the circumferential direction of the branch stent 20) with its proximal portion 22a as a base. In other implementations, 1 to 16 reinforcing ribs 212 may be arranged, and when at least two reinforcing ribs 212 are arranged, the reinforcing ribs 212 are arranged at intervals in the circumferential direction of the branch stent 20 and uniformly spaced, such that the reinforcing ribs 212 of the branch stent 20 are evenly distributed in the circumferential direction.

In this implementation, the reinforcing rib 212 is a flexible reinforcing rib 212. This can ensure that the branch stent 20 moves downward when the second end 20b of the branch stent 20 is subjected to small force. In addition, after the support section 22 moves downward, when the support section 22 is subjected to lateral force and tilts toward one side, the reinforcing rib 212 on the stressed side needs to partially return to a non-folded status to support its "tilt" shape, as shown in FIG. 5 to FIG. 6 in combination with FIG. 1. Therefore, the flexible reinforcing rib 212 can allow the support section 22 to tilt toward any side in the circumferential direction and swing and allow a more flexible swing motion, that is, the shape, supporting performance, or flexibility of the reinforcing rib 212 directly affects the smoothness and flexibility of the swing motion of the support section 22.

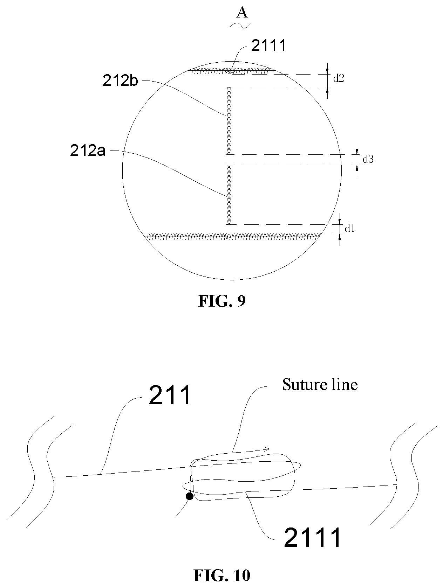

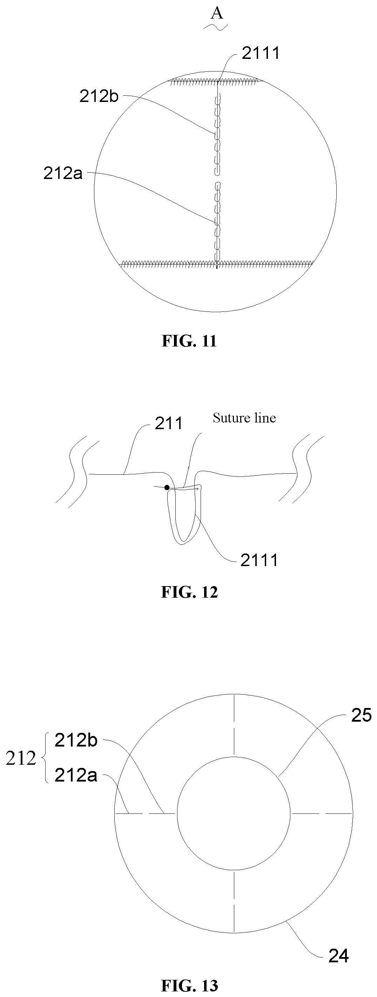

The reinforcing ribs 212 may have a "chain-like" discontinuous point structure or discontinuous segment structure. In one implementation, a suture line is sutured on the connecting film 211 in a direction from the first end 20a to the second end 20b, as shown in FIG. 7 to FIG. 8, and the suture line forms one of the reinforcing ribs 212. When the suture line is sutured, a similar discontinuous point structure or discontinuous segment structure is generally formed on any sutured side. Since the suture line is relatively soft, when the suture line is configured to suture, even if the suture line is sutured continuously or sutured back and forth for a few turns, it still has certain flexibility. In other implementations, the connecting film 211 includes a folded portion 2111; by thermally laminating the folded portion 2111 together, the folded portion 2111 forms a ridge-shaped reinforcing rib 212; the folded portion 2111 may also be thermally laminated onto a surface of an adjacent connecting film 211 directly; or the folded portion 2111 is not formed in a thermal lamination manner, but a suture line and the folded portion 2111 are sutured into a whole to form a ridge-shaped reinforcing rib 212, as shown in FIG. 9 to FIG. 12. During suturing, the suture line may be allowed to be routed around the folded portion 2111, and the folded portion 2111 is sutured onto the connecting film 211, as shown in FIG. 10; the suture line may also be sutured onto a base of the folded portion 2111 and is routed over and around the folded portion 2111, to enable the folded portion 2111 to be arranged on the connecting film 211 in a ridge shape, as shown in FIG. 12; and a specific suturing mode of the suture line and whether it is continuous are not limited, and the reinforcing rib 212 is only required to extend on the connecting film 211 in the direction from the first end 20a to the second end 20b to form a ridge-like non-rigid and rotatable reinforcing support. In addition, an extension line where the formed ridge is located does not need to be in a same plane with a tubular central axis of the branch stent, but only needs to extend in the direction from the first end to the second end, and may be a straight line, a curve, or a helix, as long as the reinforcing rib can rotate toward the main stent relative to the first end to drive the support section to move downward when the branch stent is subjected to force.

In other implementations, as shown in FIG. 2a, the reinforcing rib 212 may further include a tapered small corrugated ring 2123, and a wave height h of the tapered small corrugated ring 2123 and a wave height H of the support corrugated ring 222 satisfy: H/5≤h≤H/2. The tapered small corrugated ring includes a large head end and a small head end; one end, close to the support section, of the tapered small corrugated ring 2123 is the small head end; and one end, away from the support section, of the tapered small corrugated ring 2123 is the large head end. A corrugated ring diameter of one end, close to the support section 22, of the tapered small corrugated ring 2123 is smaller than a corrugated ring diameter of one end away from the support section 22, that is, the corrugated ring diameter of the small head end is smaller than the corrugated ring diameter of the large head end, and the wave height h of the tapered small corrugated ring 2123 is smaller than the wave height H of the support section 222. When the second end 20b of the branch stent 20 or the side edge of the branch stent 20 is subjected to force, the reinforcing rib 212 provides support for the connecting film 211, and the reinforcing rib 212 is liable to rotate relative to the first end 20a to drive the support section 22 to at least partially move downward into the adjustment section 21. In addition, a condition that the connecting film 211 is too soft to provide support in the axial direction of the branch stent 20 to cause stacking of the connecting film 211 in the axial direction is prevented. If the connecting film 211 of the adjustment section 21 is stacked in the axial direction of the branch stent 20, during the construction of a branch guidewire, the stacked connecting film 211 tends to impede the advancement of the branch guidewire.

In other implementations, the reinforcing rib 212 may also be a combination including a tapered small corrugated ring 2123 and a flexible reinforcing rib 2124, and a wave height h of the tapered small corrugated ring 2123 and a wave height H of the support corrugated ring 222 satisfy: H/5≤h≤H/2. As shown in FIG. 2b, the reinforcing rib 212 includes a tapered small corrugated ring 2123 and four flexible reinforcing ribs 2124, the tapered small corrugated ring 2123 is arranged between the flexible reinforcing ribs 2124 and the first end 20a, the four flexible reinforcing ribs 2124 are arranged between the tapered small corrugated ring 2123 and the support corrugated ring 222 in the axial direction of the branch stent, and the four flexible reinforcing ribs are arranged at intervals in the circumferential direction of the branch stent 20, and are uniformly spaced, such that the flexible reinforcing ribs 2124 of the branch stent 20 are evenly distributed in the circumferential direction of the connecting film between the tapered small corrugated ring 2123 and the support corrugated ring 222. For a configuration manner of the flexible reinforcing ribs 2124, refer to the above configuration manner of the ridge-shaped reinforcing rib formed through suturing. The tapered small corrugated ring 2123 and the flexible reinforcing ribs 2124 are combined, and the flexible reinforcing ribs 2124 are arranged between the tapered small corrugated ring 2123 and the support corrugated ring 222. This can ensure an unfolded degree of the connecting film at a connection end of the branch stent 20 and the main stent 10, while the flexible reinforcing ribs 2124 are close to the support corrugated ring 222, and the support section is more easily driven to move downward when the branch stent is subjected to force. In addition, a condition that the connecting film 211 is too soft to provide support in the axial direction of the branch stent 20 to cause stacking of the connecting film 211 in the axial direction is prevented, to avoid impeding the advancement of a branch guidewire.

In other implementations, as shown in FIG. 7, a circular truncated cone-like side edge of the adjustment section 21 may be an arc transition, and the arc is recessed toward an inner side of the branch stent 20, such that the reinforcing rib 212 extending on the connecting film 211 of the adjustment section 21 is also recessed toward the inner side of the branch stent 20, and is relatively raised toward an outer side of the branch stent 20. When receiving same external force, the reinforcing rib 212 follows an arc transition of the connecting film 211 and is recessed toward the inner side of the branch stent 20, which facilitates downward folding of the adjustment section 21.

In this implementation, as shown in FIG. 8, one of the reinforcing ribs 212 is a whole discontinuous point structure or discontinuous segment structure in its extension direction, and the reinforcing rib 212 includes a first end portion 2121 and a second end portion 2122, the first end portion 2121 is close to the first end 20a of the branch stent 20, the second end portion 2122 is close to the second end 20b of the branch stent 20, a gap d1 is formed between the first end portion 2121 and the first end 20a of the branch stent 20 or/and a gap d2 is formed between the second end portion 2122 and the support section 22, and d1 and d2 satisfy: 1 mm≤d1≤3 mm and 1 mm≤d2≤3 mm respectively, such that two ends of the reinforcing rib 212 do not extend to edges of two ends of the adjustment section 21, gaps are reserved beside two end portions of the reinforcing rib 212, and when the adjustment section 21 is folded down into a main lumen, the support section 22 moves downward into the main lumen along with the adjustment section 21, the adjustment section 21 matches the branch opening 11 to form a micro "cradle-type" structure, and the gap d1 and the gap d2 are both conducive to slight translation and swing of the support section 22 within a range of the branch opening 11 and also can increase flexibility of free swing and expand a swing range of the support section.

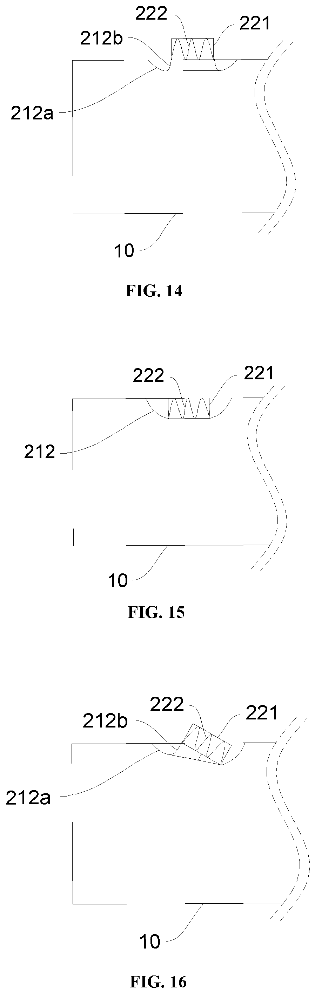

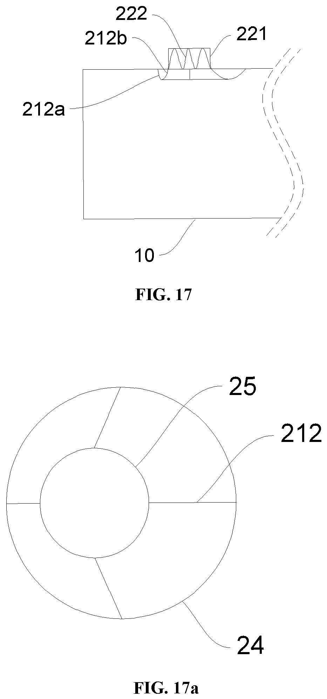

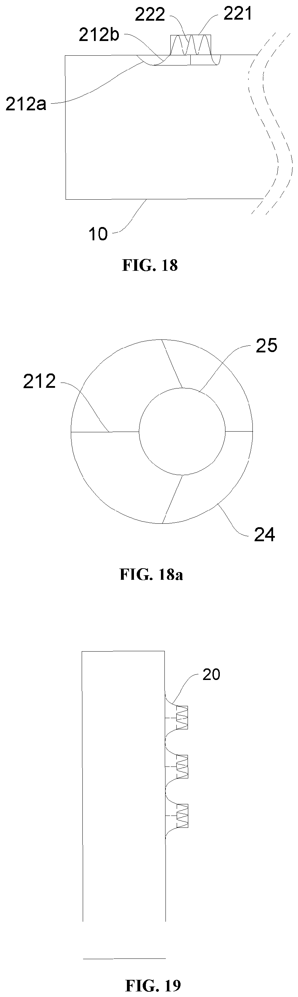

As shown in FIG. 9, a reinforcing rib 212 includes a first section 212a and a second section 212b in its extension direction, and a gap d3 is formed between the first section 212a and the second section 212b, such that the reinforcing rib 212 is divided into two sections spaced apart in its extension direction. In this implementation, a section close to the main stent 10 is the first section 212a, and a section close to the support section 22 is the second section 212b, such that the adjustment section 21 can be partially or fully folded down. When only the first section 212a is folded down and the second section 212b and the first section 212a are relatively folded, as shown in FIG. 14, the second section 212b still has a potential of further downward folding, and the second section 212b may also be folded relative to the support section 22, as shown in FIG. 15. When only the first section 212a is folded down, the second section 212b provides a certain height for the support section 22, such that downward folding of the reinforcing rib 212 does not necessarily drive the support section 22 to move downward into the lumen of the main stent 10, and when the support section 22 tilts relative to the second section 212b, the support section can tilt at a larger angle, as shown in FIG. 16. As shown in FIG. 17 to FIG. 17a and FIG. 18 to FIG. 18a, the first section 212a matches the branch opening 11 to form a micro "cradle-type" structure, such that the support section can translate and swing in the circumferential direction of the branch stent 20, and in combination with the potential of downward folding of the second section 212b, the swing flexibility of the branch stent 20 in all directions can be further enhanced.

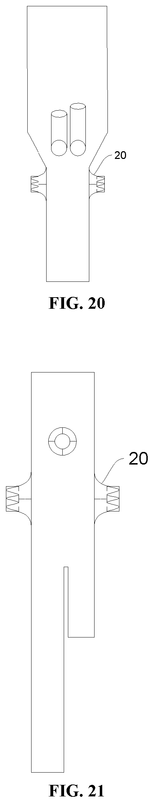

The covered stent 100 provided by the present application may be an aortic arch stent (as shown in FIG. 19), a thoracoabdominal main stent (as shown in FIG. 20), an abdominal main stent (as shown in FIG. 21), or an iliac bifurcation that needs a branch, and the branch stent 20 may be configured as the above-described structures to be applied in an aortic arch partial branch, a thoracoabdominal main branch, an abdominal main branch, and an iliac bifurcation, to form a flexibly swinging branch stent 20 to facilitate branch alignment. In addition, because the adjustment section 21 is folded down into an aortic lumen to drive the support section 22 to move downward, and the support section 22 can flexibly wing relative to the circumferential direction of the branch stent 20, when the covered stent 100 is implanted, the branch vessel opening is only required to correspond to a range of the branch opening 11 of the main stent 10. Therefore, alignment accuracy of the branch stent 20 and the branch vessel opening may be not required strictly. An opening direction (inclination angle) of the second end 20b of the branch stent 20 and an extension length of the branch stent 20 may also be flexibly adjusted according to an angle of a branch artery to be reconstructed; especially for patients with dissecting aneurysm, when a true lumen is narrow, the branch stent 20 can move downward into the main stent 10 or even be flush with a side face of the main stent 10, to avoid a condition that the branch stent is pressed due to narrow space of the true lumen of the aorta to affect the construction of the branch guidewire and blood flow in a branch.

The technical features of the above-described embodiments can be arbitrarily combined, and all possible combinations of the technical features of the above-described embodiments are described to make the description concise, but as long as no contradiction is present between the combinations of the technical features, they should be considered to fall within the scope of the present specification.

The above-described embodiments merely express a few implementations of the present application, and the description thereof is relatively specific and detailed, but should not be construed as limitations to the scope of the patent application. It should be pointed out that for those of ordinary skill in the art, several modifications and improvements can be made without departing from the concept of the present application, and these modifications and improvements all fall within the scope of protection of the present application. Therefore, the protection scope of the present application should be defined by the attached claims.

## Claims

1. A covered stent, comprising a main stent and a branch stent, wherein the main stent comprises a branch opening communicated with the branch stent; the branch stent comprises a first end and a second end which are opposite to each other, and the first end is connected to the branch opening; and the branch stent comprises an adjustment section close to the first end and a support section close to the second end, and the adjustment section comprises a reinforcing rib to allow the support section to be driven to move downward when the branch stent is subjected to force.

2. The covered stent according to claim 1, wherein the adjustment section further comprises a connecting film which connects the branch opening and the support section, the reinforcing rib is arranged on the connecting film, and the reinforcing rib is a flexible reinforcing rib.

3. The covered stent according to claim 2, wherein the reinforcing rib has a "chain-like" discontinuous point structure or discontinuous segment structure.

4. The covered stent according to claim 1, wherein the reinforcing rib comprises a first section and a second section in an extension direction of the reinforcing rib, and a gap is formed between the first section and the second section.

5. The covered stent according to claim 2, wherein the reinforcing rib comprises a suture line sutured along the connecting film;
alternatively, the connecting film comprises a folded portion, and the folded portion is thermally laminated to form the reinforcing rib; and
alternatively, the connecting film comprises a folded portion, the reinforcing rib comprises the folded portion and a suture line, and the suture line is integrally sutured along the folded portion to form the reinforcing rib.

6. The covered stent according to claim 1, wherein at least two reinforcing ribs are arranged at intervals in a circumferential direction of the branch stent.

7. The covered stent according to claim 1, wherein the branch stent comprises a first circumferential support member arranged in a circumferential direction of the branch stent, and the first circumferential support member is arranged at a connection portion between the adjustment section and the support section.

8. The covered stent according to claim 7, wherein the branch stent further comprises a second circumferential support member and a third circumferential support member, the second circumferential support member is arranged at the first end, and the third circumferential support member is arranged at the second end.

9. The covered stent according to claim 8, wherein a diameter of the first circumferential support member is defined as D1, a diameter of the second circumferential support member is defined as D2, a diameter of the third circumferential support member is defined as D3, and D1, D2, and D3 satisfy: D1≤D3<D2; and
alternatively, D1, D2, and D3 satisfy: D1≤D3, and 1.5D1≤D2≤3D1.

10. The covered stent according to any one of claims 1 to 9, wherein the reinforcing rib comprises a first end portion and a second end portion, the first end portion is close to the first end, the second end portion is close to the second end, a gap is formed between the first end portion and the first end of the branch stent, or/and a gap is formed between the second end portion and the adjustment section.

11. The covered stent according to claim 1, wherein the support section comprises a support covering film and a support corrugated ring, the support covering film covers the support corrugated ring, and the reinforcing rib comprises a tapered small corrugated ring, and a wave height h of the tapered small corrugated ring and a wave height H of the support corrugated ring satisfy: H/5≤h≤H/2.

12. The covered stent according to claim 1, wherein the reinforcing rib comprises a tapered small corrugated ring and a flexible reinforcing rib, and the tapered small corrugated ring is arranged between the flexible reinforcing rib and the first end.
